# EUROPÄISCHE PATENTANMELDUNG

(11) **EP 0 913 168 A1**
(43) Veröffentlichungstag der Anmeldung: **06.05.1999**
(21) Anmeldenummer: 97119171.3
(22) Anmeldetag: 03.11.1997
(51) Int. Cl.: A61N 5/00, A61N 1/14, A61N 1/30

(54) **Gerät und Verfahren zur Umleitung von Ionenfeldern**

(71) Anmelder: Gundlich, Heinz, Dipl.-Ing., 73422 Aalen (DE)
(72) Erfinder: Gundlich, Heinz, Dipl.-Ing., 73422 Aalen (DE)
(74) Vertreter: Einsel, Martin, Dipl.-Phys.

(57) **Zusammenfassung**

Bei einem Gerät (1) zur Umleitung von Ionenfeldern aus einem Körper oder Gegenstand in einen getrennt davon aufgestellten externen Speicher (2) weist das Gerät einen ummantelten Hohlkörper (10) auf. Der Hohlkörper ist zweifach umhült, und die innere Hülle (12) ist ein Mantel aus einem elektrisch leitenden Material und wirkt als Senkenteil für Ionenfelder, und die äußere Hülle (13) besteht im wesentlichen aus einem Trägermittel und wirkt als Emittent von Ionenfeldern. Eine Verbindung (30) zwischen Hohlkörper (10) und externem Speicher (2) ist vorgesehen.

## Beschreibung

Die Erfindung betrifft ein Gerät und ein Verfahren zum Umleiten von Ionenfeldern aus einem Körper oder einem Gegenstand in einen getrennt davon vorgesehenen, externen Speicher.

Aus der DE 44 14 340 A1 ist ein Verfahren zur Messung und Übertragung pathogener Mikrowellenenergien und ein Gerät zur Durchführung des Verfahrens bekannt. Dabei wird in das von einer Energiequelle ausgehende Mikrowellenfeld ein im wesentlichen rotationssymmetrischer Hohlkörper mit einer Längsachse und einer relativ zur Länge der Längsachse kleineren Abmessung des mittleren Durchmessers eingefügt. Die Längsachse des Hohlkörpers wird senkrecht zu einer den Hohlkörper und die Energiequelle verbindenden Linie eingestellt. Größe, Füllung und Wandung des Hohlkörpers sind so bemessen, daß ein größerer Teil der die Wandung des Körpers durchdringenden Mikrowellenenergie im Hohlkörper in Richtung seiner Längsachse umgelenkt wird und in dieser Richtung den Hohlkörper verläßt, und daß ein kleinerer Teil der die Wandung des Hohlkörpers durchdringenden Mikrowellenenergie den Hohlkörper nach Durchdringung auch der gegenüberliegenden Wandung in Eintrittsrichtung wieder verläßt. Das Gerät zur Durchführung dieses Verfahrens weist daher am Hohlkörper seitlich Mikrowellenquellen auf, die Speicher selektiver Mikrowellenenergien sind. Seitlich am Hohlkörper können weitere Hohlkörper angeordnet sein, welche seitlich eine oder mehrere Mikrowellenquellen aufweisen. Die Längsachsen der weiteren Hohlkörper können senkrecht auf die Längsachse des ersten Hohlkörpers gerichtet sein. Die seitlich am Hohlkörper angeordneten Mikrowellenquellen sind z.B. Nosoden. Die Energie der vom Hohlkörper gebündelt zur Quelle gerichteten Mikrowellen ist dabei insbesondere größer als die Energie der Mikrowellenquelle. Die Ummantelung des im wesentlichen rotationssymmetrischen bzw. zylindrischen, insbesondere mit kegelförmigen Bestandteilen versehenen Hohlkörpers besteht aus Papier, Textil oder einem ähnlichen Material und ist mit Mikrowellenenergie vorbestimmter Frequenzen und Polarisation aufgeladen. Als Material für die Wandung des Hohlkörpers wird in dieser Schrift Glas und Kupfer angegeben. Anstelle einer Folie, welche die Wandung des Hohlkörpers ummantelt, können auch zahlreiche Wicklungen aus Metall vorgesehen werden, die so bemessen sind, daß in ihnen ein elektrischer Strom fließen kann. Der jeweilige Mantel des Hohlkörpers ist in jedem Falle über eine Leitung, beispielsweise einen elektrisch leitenden Kupferdraht, mit einem Verbraucher oder Mikrowellenenergiespeicher, beispielsweise einem Behälter mit einer Flüssigkeit, verbunden.

Der Erfindung liegt die Aufgabe zugrunde, ein Gerät zum Umleiten von Ionenfeldern aus einem Körper oder einem Gegenstand in einen getrennt davon aufgestellten externen Speicher und ein Verfahren zum entsprechenden Umleiten zu schaffen, mittels derer Ionenfelder unbelebter sowie belebter Substanzen einem durch diese belasteten menschlichen oder tierischen Organismus entzogen werden können.

Die Aufgabe wird nach dem Oberbegriff des Anspruchs 1 durch dessen kennzeichnende Merkmale gelöst. Die Aufgabe wird auch durch ein Verfahren mit den Merkmalen nach Anspruch 12 gelöst. Weiterbildungen der Erfindung sind in den jeweiligen Unteransprüchen definiert.

Der Erfindung liegt die Erkenntnis zugrunde, das Ionen als elektrisch geladene Moleküle oder Atome bei einer Bewegung im Raum Ionenfelder erzeugen. Dies wird daraus geschlossen, daß Elektronen bei einer geradlinigen Bewegung durch den Raum magnetische Feldlinien erzeugen, die als konzentrische Kreise senkrecht zu ihrer Bewegungsrichtung stehen. Bei der Untersuchung nach eventuellen Feldeffekten, welche von bewegten Ionen verursacht werden, wurde festgestellt, daß Ionenfelder nicht nur angemessen werden können, sondern in physiologischen und pathologischen Prozessen eine entscheidende Rolle spielen. Ionenfelder sind schwächer, als Felder, welche durch Elektronen erzeugt werden, da die Ionen sich in festen Stoffen, Flüssigkeiten oder Gasen langsamer bewegen als Elektronen. Entsprechend den Erkenntnissen des Trägers des Physik-Nobelpreises 1997 bewegen sich Atome bzw. Ionen bei Zimmertemperatur mit 4000 km/Std. Ionenfelder können mittels beispielsweise Lecherantennen oder anderen Hilfsmitteln angemessen werden, wobei der Mensch dabei Teil des Meßgerätes sein kann. Solche Lecherantennen ermöglichen Messungen von Wellenlängen im Bereich von 4 mm bis 2000 mm. Nach Hertz ergibt sich die Lichtgeschwindigkeit als Produkt aus Wellenlänge und Frequenz, wodurch hier Frequenzen von 75 GHz bis 150 MHz auftreten. Zur Eichung kann beispielsweise die Resonanzfrequenz des Wasser mit 2,45 GHz (entsprechend 12,24 cm) herangezogen werden. Aufgrund der besonderen Formgebung der Lecherantennen arbeiten diese mit λ/4-Werten, wodurch sich für die Resonanzfrequenz des Wassers dann ein optimaler Ausschlag der Lecherantenne bei 3,06 cm bei der Anmessung von Ionenfeldern des Wassers ergibt.

Zur Verdeutlichung der Flußdichte und Ausdehnung von Ionenfeldern kann ein Gefäß mit Wasser mit einigen Körnern Kochsalz so versehen werden, daß die Lösung vollständig dissoziiert ist. Dadurch baut sich um das Gefäß herum ein glockenförmiges Ionenfeld auf, welches in seiner Ausdehnung abhängig ist von der Anzahl und dem Bewegungszustand der einzelnen Moleküle. Bei erhöhter Temperatur und damit erhöhter Bewegungsgeschwindigkeit der Kochsalzmoleküle vergrößert sich die räumliche Ausdehnung des Ionenfeldes. Nach Abkühlung auf die Ausgangstemperatur schrumpft das Ionenfeld auf seine vorherigen Größe zurück. Ein ähnlicher Effekt tritt auf, wenn das Wasser durch Umrühren in Bewegung gesetzt wird. Ionenfelder zeigen also eine andere Eigenschaft als beispielsweise elektrische oder magnetische oder elektromagnetische Felder.

Ionenfelder lassen sich durch hintereinander aufgereihte, einander berührende Hohlkörper deformieren, wobei jeweils das Ionenfeld hinsichtlich seiner Flußdichte hinter jedem Hohlkörper abnimmt. Dies Phänomen erklärt sich dadurch, daß seitlich in den Hohlkörper eingeleitete Ionenfeldern in dessen Längsachse umgeleitet werden. Es tritt dabei also eine Art von Fokussierung der Ionenfelder auf. Das in Richtung der Längsachse des Hohlkörpers austretende Ionenfeld weist dabei eine langgestreckte Keulenform auf.

Es hat sich gezeigt, daß Ionenfelder ein hohes Durchdringungsvermögen für nahezu alle festen, flüssigen und gasförmigen Stoffe, ebenso für Vakuum aufweisen, wobei kein wesentlicher Flußdichteverlust auftritt. Beispielsweise werden auch Metalle, Keramiken, Holz, Kunststoff und biologische Materialien von den Ionenfeldern durchdrungen. Es hat sich gezeigt, daß einige Materialien, wie beispielsweise Graphit, einen hohen Brechungsindex für Ionenfelder aufweisen.

Ionenfelder weisen eine Polarisation auf, sind also rechts- oder linksdrehend oder linearpolarisiert. Wird ein fokussiertes, polarisiertes Ionenfeld auf einen Spiegel gerichtet, so weist das austretende, gespiegelte Ionenfeld eine geänderte Polarisationsrichtung bei gleichbleibenden Frequenzen auf.

Der Erfindung liegt auch die Erkenntnis zugrunde, daß sich Ionenfelder auf magnetischen Feldlinien ausbreiten können. Wird beispielsweise ein Magnet in das sich um ein Gefäß mit Kochsalzlösung glockenförmig räumlich ausdehnende Ionenfeld eingebracht, kann das Ionenfeld des Kochsalzes entlang sämtlicher Feldlinien des Magneten angemessen werden. Dies gilt auch für einen Bereich außerhalb des glockenförmigen Ausdehnungsbereiches des Ionenfeldes um das Gefäß herum. Wird der Magnet mit einer Blechabschirmung ummantelt, können die magnetischen Feldlinien also die Blechabschirmung nicht durchdringen, können auch Ionenfelder nicht außerhalb des glockenförmigen räumlichen Ausdehnungsbereiches um das Gefäß herum gemessen werden. Als Magneten eignen sich hier zeitlich konstante, also Dauer- oder Elektromagneten, jedoch auch zeitlich veränderliche elektromagnetische Felder.

Der Erfindung liegt ebenfalls die Erkenntnis zugrunde, daß Ionenfelder auf Lichtstrahlen transportiert werden können, wie beispielsweise einem Laserstrahl. Wird ein solcher Laserstrahl durch das sich glockenförmig ausbreitende Ionenfeld über einem Gefäß ausgesandt und passiert der Laserstrahl dieses Ionenfeld, so lädt er sich mit den Ionenfeldern des beispielsweise Kochsalzes auf. Wird ein solcher Lichtstrahl oder Laserstrahl auf filmaktives Material gesandt, werden die auf diesem Lichtstrahl mirtransportierten Ionenfelder dort dauerhaft gespeichert. Eine temporäre Speicherung von Ionenfeldern tritt in den Luftmolekülen auf, beispielsweise in dem Moment, in dem sich eine glockenförmige Ausdehnung um ein mit Kochsalzlösung gefülltes Gefäß ergibt. Durch Luftverwirbelung kann dieses Ionenfeld wieder aufgelöst, d.h. neutralisiert werden. Eine rein temporäre Speicherung kann auch in einer Flüssigkeit, beispielsweise Wasser, geschehen.

Erfindungsgemäß wird die Möglichkeit der Speicherung von Ionenfeldern auf filmaktivem Material dadurch genutzt, daß mittels einer Kamera oder einem ähnlichen Gerätes von einem Körper eine Fotografie erstellt wird. Dabei gelangen Lichtstrahlen, welche zuvor den Ionenfeldbereich des Körpers passiert haben und dessen Ionenfelder transportieren, in das Objektiv der Kamera. Von dort aus werden sie auf das filmaktive Materialien der Kamera transportiert und auf diesem gespeichert. Das Objektiv aus Glas können sie dabei durchdringen.

Das erfindungsgemäß eingesetzte Gerät zum Umleiten von Ionenfeldern aus einem Körper in einen getrennt davon aufgestellten externen Speicher unter Verwendung eines umhüllten Hohlkörpers weist erfindungsgemäß zwei Hüllen auf. Die innere Hülle in Verbindung mit dem externen Speicher wirkt als Senke für Ionenfelder, wohingegen die äußere Hülle als Emittent von Ionenfelder wirkt, wobei diese Ionenfelder vorzugsweise mit den pathogenen Ionenfeldern des Körpers identisch sind. Die innere Hülle des Hohlkörpers ist vorzugsweise ein Mantel aus einem elektrisch leitenden Material, insbesondere Aluminiumfolie, und ist über ein beliebig langes, elektrisch leitendes Element, insbesondere einen Draht, mit einem Behälter mit Flüssigkeit, insbesondere Wasser, verbunden. Das Volumen des Behälters mit Wasser beträgt beispielsweise 160 l oder mehr und entspricht damit einer gefüllten Badewanne. Als Element zur Energieübertragung wird beispielsweise ein Kupferdraht gewählt, von dem ein ca. 1m langes, blankes Kupferdrahtstück vollständig in das Wasser eintaucht.

Im Prinzip weist der Hohlkörper eine beliebige Form auf und kann aus einem beliebigen Material bestehen. Die den Hohlkörper umgebende äußere Hülle besteht jedoch im wesentlichen aus einem Trägermittel, welches vorzugsweise ein nichtleitendes Material sein kann, beispielsweise Papier oder ein anderes nicht leitendes Material, welches mit einem Trägermaterial für Ionenfelder, beispielsweise Graphit, beschichtet ist. Das Trägermittel oder insbesondere Trägermaterial ist mit Ionenfeldern, welche dem Körper entzogen werden sollen, aufgeladen. Um eine ausreichend hohe Flußdichte dieser auf dem Trägermaterial gespeicherten Ionenfelder erzielen zu können, sind diese mit erhöhter Flußdichte versehen und so fixiert, daß keine Flußdichteverluste bei längerem Gebrauch auftreten.

Besonders bevorzugt wird anstelle eines Trägermateriales aus Graphit filmaktives Material verwendet, auf dem entsprechende Ionenfelder gespeichert sind. Da sowohl Trägermaterial als auch filmaktives Material auf die innere Hülle, also den als Senke fungierenden Teil des Gerätes, einwirken, kann dieser Zweck auch von einem oder mehreren weiteren Hohlkörpern erzielt werden, der oder die auf den inneren Mantel fokussiert wird/werden. Der Zweck kann alternativ auch von einem in die Strahlungskeule des Gerätes eingebrachten weiteren Hohlkörper erfüllt werden. Eine weitere Möglichkeit besteht darin, ein magnetisches Mittel so in das von dem Körper oder Organismus emittierte Ionenfeld mit seinen Feldlinien einzubringen, daß die magnetischen Feldlinien selbiges Ionenfeld und zugleich die innere Hülle des Gerätes schneiden oder durchdringen.

Von der äußeren Hülle als Emittent werden die darin gespeicherten Ionenfelder radial in den Hohlkörper hinein emittiert. In dem Hohlkörper werden sie in Richtung der Hohlkörperachse umgelenkt und treten in Längsrichtung des Hohlkörpers als keulenartiges Ionenfeld aus diesem wieder aus. Dieses Keulenfeld wird auf einen Körper gerichtet. Trifft dabei eine Feldlinie des Ionenfeldes der äußeren Hülle des Hohlkörpers in dem Körper auf einen Bereich, der Ionenfelder enthält, welche mit denen dieser Feldlinie identisch sind, und mit gleicher Frequenz und Polarisation schwingen, wirkt diese Feldlinie wie ein Leitpfad, über den die im Körper gespeicherten Ionenfelder (pathogene Energien) abfließen können. Die abfließenden Ionenfelder gelangen in den Senkenbereich des Hohlkörpers, also in die innere Hülle. Da diese mit dem externen Speicher über das ableitende Element verbunden ist, werden die so in den Senkenbereich gelangten Ionenfelder aus diesem in den mit Wasser gefüllten Behälter als externem Speicher abgeleitet. Dadurch kann eine vollständige Entladung des entsprechenden Körperbereiches von Ionenfeldern vorgenommen werden, wobei lediglich diejenigen Ionenfelder dem Körper entzogen werden, welche im Trägermittel bzw. insbesondere Trägermaterial, filmaktiven Material etc. der äußeren Hülle gespeichert sind.

Der Entzug der Ionenfelder geschieht dabei völlig berührungslos. Bei Verwendung eines weiteren Hohlkörpers als Emittent für Ionenfelder kann dieser entweder radial oder aber aus einer Schräglage heraus auf die innere Hülle als Senkenbereich des Hohlkörpers gerichtet sein.

Im Rahmen des erfindungsgemäßen Verfahrens wird eine Prüfung auf Belastungen des Organismus oder Körpers durch verschiedene organische und anorganische Materialien beispielsweise auch auf Gifte vorgesehen. Zur Überprüfung der Belastung auf bösartige Tumore wird erfindungsgemäß die Intensität des Neurotransmitters Acetylcholin geprüft, welche bei einer bösartigen Tumorbelastung von einem Normalwert auf einen reduzierten Wert abfällt.

Zur Überprüfung der Belastung durch Mykosen wird auf das Stoffwechselverhalten von Mykosen Bezug genommen. Daher werden Stoffwechselprodukte, welche allen Mykosen gemeinsam sind, wie beispielsweise Schwefel- und Phosphorverbindungen oder Antimon, hinsichtlich ihrer eventuell erhöhten Intensität im Körper gemessen. Zur Überprüfung auf bakterielle Infektionen wird auf deren Stoffwechselverhalten, insbesondere bestimmte Stoffwechselprodukte, die allen Bakterienarten gemeinsam sind, wie beispielsweise Kobalt- und Wismutverbindungen, Bezug genommen. Im Normalfalle liegen die Konzentrationen von Kobalt- und Wismutverbindungen in einem kaum meßbarem Bereich, wodurch bei entsprechend feststellbaren Konzentrationen auf eine bakterielle Infektion geschlossen werden kann.

Zur Prüfung auf Virusbelastungen des Körpers wird die Konzentration von Interferon überprüft, da aus Abwehrgründen ein Organismus bei Virusbelastung Interferon freisetzt.

Zur Überprüfung auf Prionenbelastung werden Nosoden verwendet, die Informationen aller bereits aufgefundenen Prionen enthalten. Es eignen sich hierbei besonders Nosoden vom Typ Lepra, die sämtliche Klassen von Prionen enthalten.

Wird eine Belastung des Körpers durch Erreger wie Mykosen, Bakterien, Viren oder Prionen festgestellt, wird eine Bekämpfung dieser Erreger in zwei Schritten vorgenommen. Zunächst werden deren spezifische Ionenfelder mittels des Verfahrens wie oben beschrieben den Erregern entzogen. Diese regenerieren sich nach einer bestimmten Zeit, weswegen vorab der zweite Schritt eingeleitet wird. In diesem werden die Erreger für das Immunsystem des Körpers markiert. Dies geschieht dadurch, daß Körper und Erreger von einem Ionenfeld durchflutet werden, welches das Frequenzspektrum der Ionenfelder des Erregers in erhöhter Flußdichte enthält. Dadurch werden die Erreger verändert und für beispielsweise das Immunsystem des Körpers erkennbar und angreifbar gemacht. Das körpereigene Immunsystem kann danach die Erreger selbst bekämpfen.

Um einen bestimmten belasteten Körperbereich und die darin befindlichen Erreger durch Ionenfelder mit erhöhter Flußdichte durchfluten zu können, wird besonders bevorzugt ein Trägermittel verwendet, auf dem diese Ionenfelder in erhöhter Flußdichte gespeichert und fixiert sind. Als Trägermittel oder Trägermaterial eignet sich hier unter anderem Kochsalz, mit dem ein textiles Material, insbesondere ein Tuch, getränkt wird. Dieses textile Material kann dann den Hohlkörper des erfindungsgemäßen Gerätes ummanteln.

Die jeweilig zu wählende Zeitdauer der Durchflutung des zu behandelnden Körpers oder Körperteiles ist abhängig von der Schwere der Belastung und beträgt im wesentlichen einige Stunden.

Zur näheren Erläuterung der Erfindung werden im folgenden Ausführungsbeispiele anhand der Zeichnungen beschrieben:

Diese zeigen in:
- **Figur 1**: eine Prinzipskizze in Schnittansicht eines erfindungsgemäß aufgebauten Gerätes,
- **Figur 2**: eine Detail-Schnittansicht durch einen Teil eines erfindungsgemäß aufgebauten Hohlkörpers,
- **Figur 3**: eine Prinzipskizzse einer zweiten Ausführungsform eines erfindungsgemäßen Gerätes,
- **Figur 4**: eine Prinzipskizze einer dritten Ausführungsform eines erfindungsgemäßen Gerätes, und
- **Figur 5**: eine Prinzipskizze einer vierten Ausführungsform eines erfindungsgemäßen Gerätes.

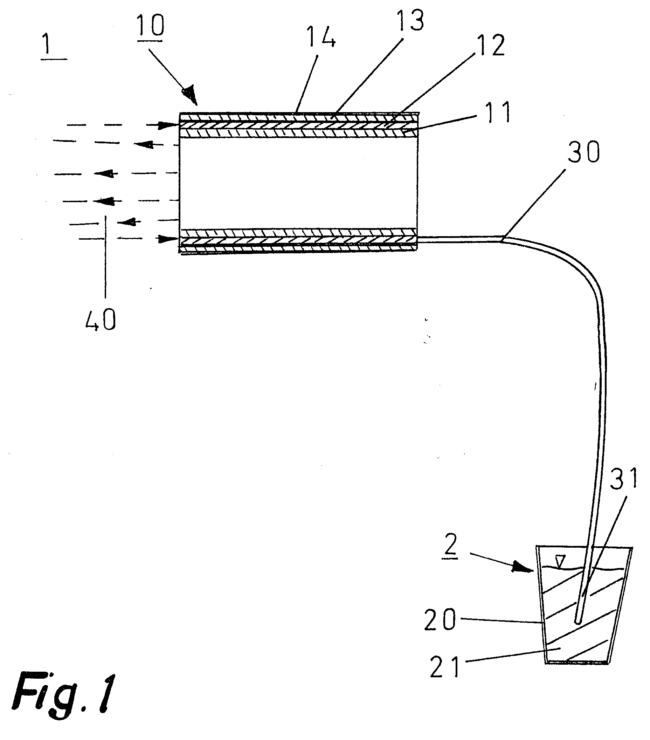

In **Figur 1** ist eine Prinzipskizze eines erfindungsgemäßen Gerätes 1 zum Umleiten von Ionenfeldern aus einem Körper oder sonstigem Substrat (in Figur 1 nicht dargestellt) in einen getrennt davon aufgestellten, externen Speicher 2 dargestellt. Das Gerät 1 weist einen Hohlkörper 10 beliebiger Formgebung auf. Im dargestellten Falle ist im wesentlichen ein zylindrischer Hohlkörper vorgesehen. Der Hohlkörper weist eine Wandung 11 auf. Er ist aus einem beliebigen Material z.B. Glas gefertigt. Die Wandung 11 des Hohlkörpers wird von einem inneren Mantel 12 umhüllt. Dieser wiederum wird von einem äußeren Mantel 13 umhüllt. Der innere Mantel ist aus einem elektrisch leitfähigen Material, insbesondere aus Aluminiumfolie, gefertigt. Er ist mit einem leitfähigen Element 30, insbesondere einem Kupferdraht, versehen. Das leitfähige Element 30 reicht zum externen Speicher 2 hin.

Der externe Speicher 2 weist ein Behältnis 20 auf. Dieses ist mit einer Flüssigkeit 21, insbesondere mit Wasser, gefüllt. Ein Teilstück 31, insbesondere ein 1m langes blankes Kupferdrahtstück, des leitfähigen Elementes 30 ist in die Flüssigkeit 21 in dem Behältnis 20 des externen Speichers 2 eingetaucht.

Der äußere Mantel 13 des Hohlkörpers 10 des Gerätes 1 ist vorzugsweise beschichtet. Auf diesem ist, wie in Figur 1 dargestellt, ein Trägermaterial 14 aufgefügt. Das Trägermaterial ist mit Ionenfeldern gespeist bzw. diese sind in hoher Flußdichte auf diesem gespeichert und fixiert. Als Ionenfelder werden gerade diese gespeichert, welche dem Körper oder Substrat entzogen werden sollen.

Von dem Hohlkörper wird ein Feld ausgesandt. Dieses ist insbesondere keulenförmig, was jedoch in Figur 1 nicht erkennbar ist. Dieses Feld, welches von dem beschichteten Trägermaterial 14 ausgeht, in Richtung der Hohlkörperachse umgelenkt ist, gelangt zu dem Körper oder Substrat, welches seinerseits zumindest in einem bestimmten Bereich entsprechende Ionenfelder emittiert. Aufgrund der höheren Flußdichte der von dem Trägermaterial, also dem Hohlkörper, ausgesandten Ionenfelder im Vergleich zu denen, welche von einem bestimmten Bereich des Körpers ausgesandt werden, werden entsprechende Ionenfelder zu dem Hohlkörper geleitet. Diese gelangen in den inneren Mantel 12 des Hohlkörpers, welcher aus dem leitfähigen Material besteht. Sie werden daher weitergeleitet zu dem leitfähigen Element 30 und über dieses in das Behältnis 20 mit der Flüssigkeit 21.

Während des Umleitens der Ionenfelder aus dem Körper in den externen Speicher kann am leitfähigen Element 30 das entsprechende Ionenfeld gemessen werden. Dies kann beispielsweise mittels einer bekannten Lecherantenne geschehen.

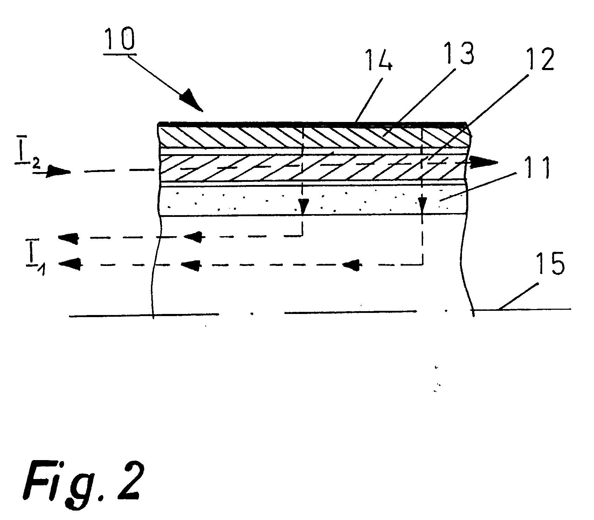

**Figur 2** zeigt einen Detailausschnitt des Hohlkörpers gemäß Figur 1. Dabei sind die einzelnen Schichten der Wandung 11 des Hohlkörpers 10, des inneren Mantels 12 sowie des äußeren Mantels 13 mit dem darauf aufgefügten Trägermaterial 14 verdeutlicht. Das in dem Trägermaterial 14 gespeicherte Ionenfeld I₁ gelangt in das Innere des Hohlkörpers 10 und wird in dessen Längsrichtung, also in Richtung seiner Längsachse 15, umgelenkt. Das Ionenfeld I₁ wird also aus dem Hohlkörper emittiert.

Von der belasteten Stelle des Körpers oder eines Substrates ausgehend gelangt das entsprechende Ionenfeld I₂ wieder zu dem Hohlkörper zurück. Es dringt in den inneren Mantel 12 des Hohlkörpers 1 ein und wird von diesem, aufgrund der Leitfähigkeit seines Materials, weitergeleitet. Dieses Ionenfeld I₂ wird also immittiert.

Als Trägermaterial 14 kann entweder entsprechend vorbehandeltes Graphit dienen. Ebenso kann aber auch filmaktives Material verwendet werden.

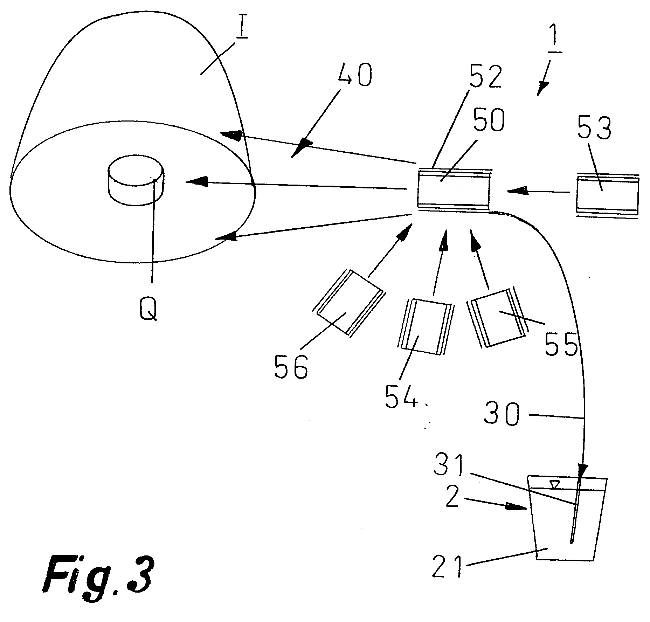

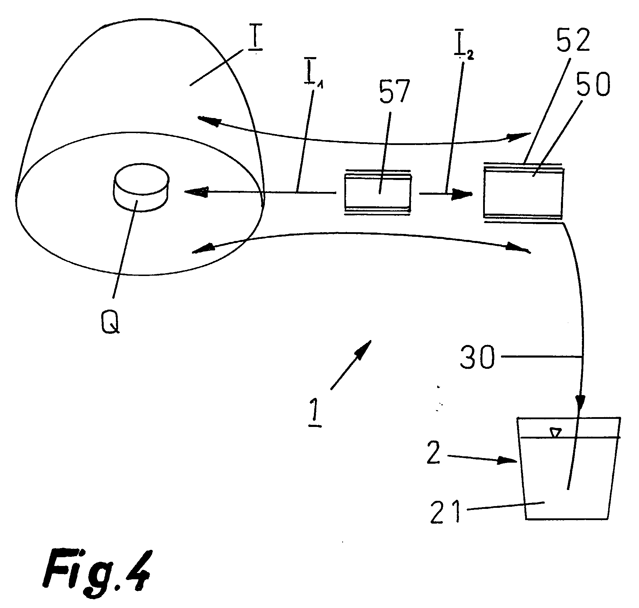

Alternativ hierzu ist es auch möglich, anstelle der Beschichtung des äußeren Mantels mit dem Trägermaterial auf dem äußeren Mantel des ersten Hohlkörpers 1 einen weiteren Hohlkörper, welcher entsprechende Ionenfeldern aussendet, aufzufügen bzw. in dessen Nähe anzuordnen. Die Fokussierachse dieses Hohlkörpers ist dann vorzugsweise in Richtung auf den inneren Mantel des Hohlkörpers gerichtet, insbesondere schräg zu diesem angestellt. Entsprechende Ausführungsformen zeigen die Figuren 3 und 4.

In **Figur 3** ist eine alternative Ausführungsform eines erfindungsgemäßen Gerätes 1 mit externem Speicher 2 dargestellt. Das Gerät weist einen Hohlkörper 50 auf, welcher auf eine Ionenfeldquelle Q gerichtet ist. Die Ionenfeldquelle Q erzeugt um sich herum ein kugelförmiges Ionenfeld I, von dem jedoch lediglich eine Hälfte dargestellt ist. Im Unterschied zu dem Hohlkörper 10 gemäß den vorigen Figuren weist der Hohlkörper 50 gemäß Figur 3 lediglich eine innere Hülle 52 auf. Die innere elektrisch leitfähige Hülle 52 ist über das leitfähige Element 30 ebenfalls mit dem externen Speicher 2 verbunden.

Anstelle eines äußeren Mantels 13 gemäß Figur 1 oder Figur 2 wird bei dieser alternativen Ausführungsform gemäß Figur 3 eine äußere Hülle durch Einbringen von äußeren Ionenfeldern geschaffen. Zu diesem Zweck sind weitere Hohlkörper 53 bis 56 auf die innere Hülle 52 des Hohlkörpers 50 ausgerichtet. Diese Hohlkörper weisen ebenfalls lediglich eine innere Hülle auf. Die weiteren Hohlkörper emittieren jeweils ein vorbestimmbares entsprechendes Ionenfeld, welches als Trägermittel und damit als Emittent von Ionenfeldern dient.

Wie bereits in den vorigen Ausführungsformen wird dadurch von dem Hohlkörper ein keulenförmiges Strahlungsfeld emittiert. Die weiteren Hohlkörper können in einem beliebigen Winkel zur inneren Hülle 52 des Hohlkörpers 50 angeordnet sein. Beispielsweise ist der weitere Hohlkörper 53 auf die Rückseite des Hohlkörpers 50 gerichtet, der weitere Hohlkörper 54 ist im wesentlichen senkrecht auf die innere Hülle 52 gerichtet, die beiden weiteren Hohlkörper 55 und 56 sind hingegen schräg zur inneren Hülle 52 gerichtet angeordnet.

Die innere Hülle 52 besteht vorzugsweise aus einem elektrisch leitfähigen Material. Beispielsweise kann zu Anschauungszwecken als Ionenfeldquelle Q eine Kochsalzlösung verwendet werden. Die inneren Hüllen der weiteren Hohlkörper 53 bis 56 enthalten dann vorzugsweise ebenfalls eine Kochsalzlösung. Sie entsprechen damit ionenmäßig dem Ionenfeld I der Quelle Q. Eine Ionenfeldumladung aus dem Ionenfeld I in den Hohlkörper 50 und von dort in den Speicher 2 kann bei beliebiger Anordnung der weiteren Hohlkörper 53 bis 56 erfolgen.

**Figur 4** zeigt eine weitere Ausführungsform des erfindungsgemäßen Gerätes, bei dem ein weiterer Hohlkörper 57 zwischen den Hohlkörper 50 und die Ionenfeldenergiequelle Q eingefügt ist. Auch bei einer solchen Anordnung erfolgt die gewünschte Ionenfeldumladung von der Ionenfeldenergiequelle Q über den Hohlkörper 50 in den externen Speicher 2.

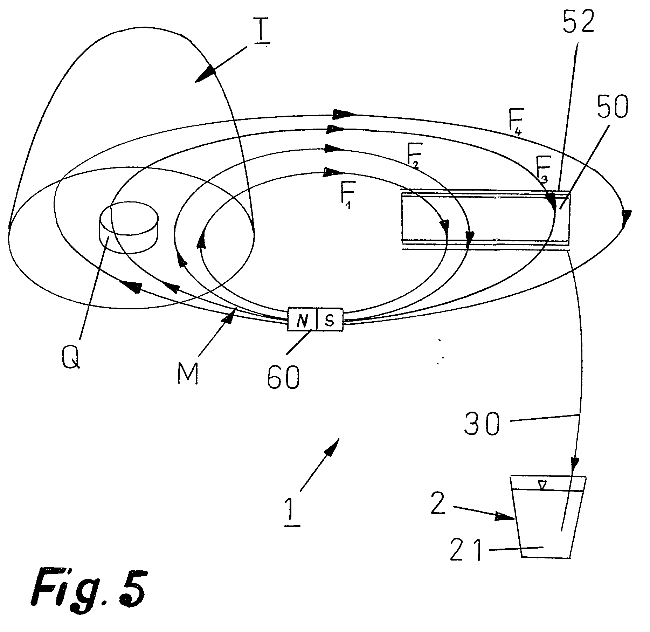

**Figur 5** zeigt eine weitere Ausführungsform eines erfindungsgemäßen Gerätes 1. Anstelle der weiteren Hohlkörper, wie sie in den Figuren 3 und 4 dargestellt sind, wird in dieser Ausführungsvariante ein magnetisches Mittel 60 beispielsweise in Form eines Dauer- oder Elektromagneten oder aber eines magnetischen Feldes verwendet. Das magnetische Mittel 60 ist so zwischen Ionenfeld I und Hohlkörper 50 eingefügt, daß das Magnetfeld M mit seinen Feldlinien F₁ bis F₃ sowohl das Ionenfeld I als auch den Hohlkörper im Bereich seiner inneren Hülle 52 schneidet. Anstelle eines Dauermagnetfeldes sind auch Gleich- oder Wechselstrommagnetfelder oder elektromagnetische Schwingungsfelder möglich. Die Ionenfelder, welche von der Ionenfeldquelle Q emittiert werden, welche beispielsweise eine erkrankte Stelle eines Körpers oder Organismus ist, werden über die geschlossenen Feldlinien zu dem Hohlkörper bzw. zu dessen innerer Hülle 52 getragen. Von dort werden sie in den externen Speicher 2 abgeleitet.

Bei den im vorstehenden genannten Ausführungsvarianten kann jeweils ein tragender Hohlkörper, also eine Wandung des Hohlkörpers, für die als Senkenteil wirkende innere Hülle entfallen, sofern die äußere Hülle eine Hohlkörperform aufweist. Ebenso kann eine Wandung eines Hohlkörpers als tragendes Element für die als Emittent wirkende äußere Hülle entfallen, sofern das äußere Trägermittel als Trägermaterial die Form eines Hohlkörpers aufweist.

Anstelle der Ausführungsvarianten gemäß Figur 3 bis Figur 5 könnte auch die innere Hülle als ein als Senkenteil wirkendes Element bis hinein in das Ionenfeld I geführt werden. Auf ein Trägermittel als koppelnden Emittenten könnte dann vollständig verzichtet werden. Im praktischen Gebrauch zum Entzug von pathogenen Energien aus einem Organismus stellt diese Ausführungsvariante jedoch eine weniger wichtige dar, da mit dieser Ausführungsvariante auch die physiologischen Energien des Organismus entzogen werden können.

### Bezugszeichenliste

- 1: Gerät
- 2: externer Speicher
- 10: Hohlkörper
- 11: Wandung
- 12: innerer Mantel
- 13: äußerer Mantel
- 14: Trägermaterial
- 15: Längsachse
- 20: Behältnis
- 21: Flüssigkeit
- 30: leitfähiges Element
- 31: Teilstück
- 40: Strahlungsfeld
- 50: Hohlkörper
- 52: innere Hülle
- 53: weiterer Hohlkörper
- 54: weiterer Hohlkörper
- 55: weiterer Hohlkörper
- 56: weiterer Hohlkörper
- 57: weiterer Hohlkörper
- 60: magnetisches Mittel
- I₁: emittiertes Ionenfeld
- I₂: immittiertes Ionenfeld
- Q: Ionenfeld-Quelle
- I: Ionenfeld
- M: Magnetfeld
- F₁: Feldlinien
- F₂: Feldlinien
- F₃: Feldlinien
- F₄: Feldlinien

## Patentansprüche

1. Gerät zur Umleitung von Ionenfeldern aus einem Körper oder Gegenstand in einen getrennt davon aufgestellten externen Speicher, wobei das Gerät einen ummantelten Hohlkörper aufweist,
**dadurch gekennzeichnet,**
**daß** der Hohlkörper zweifach umhüllt ist,
**daß** die innere Hülle ein Mantel aus einem elektrisch leitenden Material ist und als Senkenteil für Ionenfelder wirkt und die äußere Hülle im wesentlichen aus einem Trägermittel besteht und als Emittent von Ionenfeldern wirkt, und
**daß** eine Verbindung zwischen Hohlkörper und externem Speicher vorgesehen ist.

2. Gerät nach Anspruch 1,
**dadurch gekennzeichnet,**
**daß** das elektrisch leitende Material des inneren Mantels eine Aluminiumfolie ist und daß der innere Mantel über ein elektrisch leitendes Element mit einem Wasserreservoir als externem Speicher verbunden ist.

3. Gerät nach Anspruch 1 oder 2,
**dadurch gekennzeichnet,**
**daß** die äußere Hülle ein Mantel aus einem nichtleitenden Material ist und mit einem Trägermaterial als Trägermittel beschichtet ist.

4. Gerät nach Anspruch 1 oder 2,
**dadurch gekennzeichnet,**
**daß** die äußere Hülle von einem oder mehreren äußeren Ionenfeldern gebildet ist, die das Trägermittel bilden.

5. Gerät nach Anspruch 4,
**dadurch gekennzeichnet,**
**daß** die äußeren Ionenfelder durch einen oder mehrere weitere, auf die innere Hülle des Hohlkörpers gerichtete, mit einem Trägermittel versehene Hohlkörper oder durch zumindest einen in Linie mit dem Hohlkörper ausgerichteten, in dessen Strahlungskeule eingebrachten weiteren Hohlkörper oder durch ein durch ein magnetisches Mittel übertragenes Ionenfeld erzeugbar sind.

6. Gerät nach Anspruch 5,
**dadurch gekennzeichnet,**
**daß** das magnetisches Mittel so angeordnet ist, daß die magnetischen Feldlinien sowohl das von dem Gegenstand oder Körper ausgesandte Ionenfeld als auch die innere Hülle des Hohlkörpers schneiden oder durchdringen.

7. Gerät nach Anspruch 5 oder 6,
**dadurch gekennzeichnet,**
**daß** das magnetische Mittel ein Dauermagnet oder ein Elektromagnet oder ein elektromagnetisches Feld ist.

8. Gerät nach einem der vorstehenden Ansprüche,
**dadurch gekennzeichnet,**
**daß** das Trägermittel der äußeren Hülle mit pathogenen Ionenfeldern, welche denen entsprechen, welche dem Körper entzogen werden sollen, aufgeladen ist, wobei vor der Speicherung der Ionenfelder im Trägermittel eine Erhöhung von deren Flußdichte vorsehbar ist und die Ionenfelder so fixiert sind, daß kein Verlust der Ionenfeld-Flußdichte auftritt.

9. Gerät nach Anspruch 8,
**dadurch gekennzeichnet,**
**daß** die in dem Trägermittel gespeicherten und fixierten Ionenfelder vor Speicherung und Fixierung mit erhöhten Ionenfeld-Flußdichten versehen sind, wobei sie den Ionenfeldern entsprechen, die dem Körper entzogen werden sollen.

10. Gerät nach einem der vorstehenden Ansprüche,
**dadurch gekennzeichnet,**
**daß** das Trägermittel auf ein Textilmaterial aufgebracht ist und dieses als äußerer Mantel des Hohlkörpers dient.

11. Gerät nach Anspruch 1,
**dadurch gekennzeichnet,**
**daß** die äußere Hülle ein Mantel aus filmaktivem Material ist, auf welchem aus dem Körper zu entladende Ionenfelder gespeichert und fixiert sind.

12. Verfahren zum Umleiten von Ionenfeldern aus einem Körper oder Gegenstand in einen entfernt davon aufgestellten externen Speicher, insbesondere unter Verwendung eines Gerätes gemäß Anspruch 1 bis 11, **dadurch gekennzeichnet,**
**daß** die dem Organismus oder Körper zu entziehenden Ionenfelder ermittelt werden,
**daß** Ionenfelder dem Körper oder Gegenstand dadurch entzogen werden, daß Erreger oder Organismus bzw. Körper, welche Ionenfelder emittieren, von einem Ionenfeld durchflutet werden, das Frequenzspektren der entsprechenden Ionenfelder mit erhöhter Flußdichte enthält.

13. Verfahren nach Anspruch 12,
**dadurch gekennzeichnet,**
**daß** der Körper als Ganzes oder in Abschnitten für eine vorbestimmte Zeitspanne von den Ionenfeldern mit erhöhter Flußdichte durchflutet wird.

14. Verfahren nach Anspruch 12 oder 13,
**dadurch gekennzeichnet,**
**daß** zum Ermitteln des entsprechenden Ionenfeldtyps, welcher den Organismus oder Körper belastet und welcher mit erhöhter Flußdichte eingesetzt werden soll, eine Prüfung auf Belastung durch Gifte, Tumore, Mykosen, bakterielle Infektionen, Virusbelastungen und/oder Prionenbelastungen geprüft wird.

15. Verfahren nach Anspruch 14,
**dadurch gekennzeichnet,**
**daß** bei einer Prüfung auf Belastung durch bösartige Tumore die Intensität des Neurotransmitters Acetylcholin geprüft wird,
**daß** zur Prüfung auf Belastung durch Mykosen auf deren Stoffwechselprodukte, insbesondere auf Schwefel- und Phosphorverbindungen oder auf Antimon geprüft wird,
**daß** zur Prüfung auf bakterielle Infektionen auf deren Stoffwechselprodukte, insbesondere auf Kobalt- und Wismutverbindungen, geprüft wird,
**daß** zur Prüfung auf Virusbelastungen auf freigesetztes Interferon geprüft wird,
**daß** zur Prüfung auf Prionenbelastung eine Nosode vom Typ Lepra, die sämtliche Klassen von Prionen enthält, verwendet wird.

16. Verfahren nach einem der Ansprüche 12 bis 15,
**dadurch gekennzeichnet,**
**daß** die den Körper oder Organismus belastenden Ionenfelder auf filmaktivem Material gespeichert werden, wobei Ionenfeld-Flußdichte-Messungen der Belastungen anhand des filmaktiven Materials vorgenommen werden.

17. Verfahren nach einem der Ansprüche 12 bis 16,
**dadurch gekennzeichnet,**
**daß** ein als Senke für Ionenfelder wirkender Teilbereich eines Gerätes, insbesondere ein innerer Mantel eines Hohlkörpers, von Ionenfeldern durchflutet wird.

18. Verfahren nach einem der Ansprüche 12 bis 17,
**dadurch gekennzeichnet,**
**daß** die von dem Körper oder Gegenstand ausgesandten, umzuleitenden Ionenfelder durch geeignetes Positionieren eines magnetischen Mittels auf dessen ausgesandten Feldlinien auf die innere Hülle des Hohlkörpers geleitet werden.
